# EUROPEAN PATENT SPECIFICATION

(11) **EP 2 892 582 B1**
(45) Date of publication and mention of the grant of the patent: **13.11.2019**
(21) Application number: 13835216.6
(22) Date of filing: 10.09.2013
(51) Int. Cl.: A61M 1/10, A61B 5/021, A61B 5/026

(54) **INTRAVENOUS ACCESS DEVICE HAVING INTEGRATED HEMODYNAMIC RESUSCITATION SYSTEM.**
INTRAVENÖSE ZUGANGSVORRICHTUNG MIT EINEM INTEGRIERTEN HÄMODYNAMISCHEN WIEDERBELEBUNGSSYSTEM.
DISPOSITIF D'ACCÈS INTRAVEINEUX COMPORTANT UN SYSTÈME DE RÉANIMATION HÉMODYNAMIQUE INTÉGRÉ.

(30) Priority: 10.09.2012 US 201261698790 P
(43) Date of publication of application: 15.07.2015
(73) Proprietor: Vanderbilt University, Nashville, TN 37240 (US); The United States as Represented by The Department of Veterans Affairs, Washington, DC 20420 (US)
(72) Inventor: SEXTON, Kevin, Nashville, Tennessee 37721 (US); EAGLE, Susan, Nashville, Tennessee 37212 (US); HOCKING, Kyle, Alpharetta, Georgia 30022 (US); BAUDENBACHER, Franz, Franklin, Tennessee 37069 (US); BROPHY, Colleen, Nashville, Tennessee 37209 (US); BOYER, Richard, Nashville, Tennessee 37206 (US)
(74) Representative: Gill Jennings & Every LLP
(86) International application number: PCT/US2013/058992
(87) International publication number: WO 2014/040045

(56) References cited:
- WO-A2-02/24053
- WO-A2-02/091910
- US-A- 5 643 302
- US-A1- 2003 004 492
- US-A1- 2007 225 614
- US-A1- 2008 262 418
- US-A1- 2011 087 301
- US-B2- 7 927 270

## Description

### Technical Field

The present disclosure relates generally to hemodynamic resuscitation, and more particularly to a hemodynamic resuscitation system that is at least partially integrated with an intravenous access device.

### Background

In the United States, traumatic injury is responsible for one human death every three minutes, accounting for approximately 51 % of all deaths in persons aged 1-44 years. One of the leading reasons for these deaths is the lack of adequate early resuscitation measures. Generally, early resuscitative measures relate to restoring normal vital signs in the patient, including heart rate, blood pressure, and urine output; however, up to 85% of trauma patients that exhibit normal vital signs also show evidence of compensated shock, a major source of morbidity and mortality in trauma patients when not properly treated.

Generally, compensated shock is due to inadequate tissue perfusion (measured in terms of hemodynamic status), which can be improved through hemodynamic resuscitation. Hemodynamic resuscitation can be used for other conditions, such as congestive heart failure or kidney failure, where hemodynamic status is important. Non-invasive medical devices do exist to estimate a patient's hemodynamic status; however, the implementation of these devices as early resuscitative measures requires significant modifications to the existing healthcare protocols. Changing existing healthcare protocols historically has met with resistance. Additionally, these devices are strictly physiologic monitors that cannot control delivery of therapies to the patient, leaving open the possibility of improper treatment of compensated shock. US2003/004492 A1 discloses a method and apparatus for controlling blood volume an hydration and for indicating resuscitation status of a patient using peripheral venous pressure as a hemodynamic parameter. US2008/262418 A1 discloses an automated therapy system having an infusion catheter; a sensor adapted to sense a patient parameter; and a controller communicating with the sensor and programmed to control flow output from the infusion catheter into a patient based on the patient parameter without removing fluid from the patient.

### Summary

In one aspect, the present disclosure includes a system for hemodynamic resuscitation. The system includes an occlusion device configured to occlude a peripheral vein; an intravenous access device having a pressure sensor element configured to detect a peripheral venous pressure value in response to the occlusion of a peripheral vein by the occlusion device and to interface with an external fluid source for fluid delivery to the vein; and a controller device, coupled to the pressure sensor element and the external fluid source, configured to: receive a signal comprising data related to the peripheral venous pressure value, process the signal to determine a hemodynamic parameter by comparing wavelets within the signal to a template wavelet function, and generate a resuscitation score based on the hemodynamic parameter, wherein an amount of the resuscitative fluid is delivered to the peripheral vein from the fluid source through the intravenous access device based on the resuscitation score.

In another aspect, the present disclosure includes a non-transitory computer-readable device storing instructions executable by an associated processor to perform operations that facilitate hemodynamic resuscitation. The operations include processing a peripheral venous pressure value detected by a pressure sensor within an intravenous access device to achieve a hemodynamic parameter by comparing wavelets within the signal to a template wavelet function. The operations also include generating a resuscitation score based on the hemodynamic parameter. The operations further include signaling a component of the intravenous access device to allow an amount of fluid to be delivered from an external fluid source to the vein, based on the resuscitation score.

### Brief Description of the Drawings

The foregoing and other features of the present disclosure will become apparent to those skilled in the art to which the present disclosure relates upon reading the following description with reference to the accompanying drawings, in which:
FIG. 1 is a schematic illustration of an example hemodynamic resuscitation system in accordance with one aspect of the present disclosure;
FIG. 2 is a schematic illustration of an example intravenous configuration that can be utilized within the hemodynamic resuscitation system of FIG. 1;
FIG. 3 is a schematic illustration of an example external configuration that can be utilized within the hemodynamic resuscitation system of FIG. 1;
FIG. 4 is a schematic illustration of an example controller device that can be utilized within the system of FIG. 1; and
FIG 5 is schematic process flow diagram of an example method that facilitates hemodynamic resuscitation.

### Detailed Description

The present invention generally relates to hemodynamic resuscitation. When used herein, the term "hemodynamic" generally refers to blood movement, and "hemodynamic resuscitation" generally refers to increasing blood movement (or blood pressure) in a patient experiencing symptoms of compensated shock (e.g., based on a "hemodynamic score" or "resuscitation score"). In addition to compensated shock, the present invention relates to all applications where hemodynamic status of the patient is critical. An example of an application where the hemodynamic status of the patient is critical is congestive heart failure (CHF). With CHF, an intravascular volume status that is too high can cause CHF exacerbations, while an intravascular volume status that is too low can cause pre-renal acute kidney injury/failure (e.g., from diuretic use or third spacing). When applications of the present invention are described herein as referring to "compensated shock," it will be understood that the applications can also relate to other applications where the hemodynamic status of the patient is critical (e.g., CHF).

Hemodynamic resuscitation as described herein can be accomplished via a hemodynamic resuscitation system that includes an intravenous access device having a pressure sensor element configured to detect a peripheral venous pressure value in response to an occlusion of a peripheral vein. The system also includes a controller device that is configured to receive a signal from the pressure sensor comprising the peripheral venous pressure value, to process the signal to determine a hemodynamic parameter (e.g., a parameter that correlates to left ventricle end diastolic volume or stroke volume or another volume that has evidence of compensated shock). Based on the peripheral venous pressure value, and to generate a resuscitation score based on the hemodynamic parameter.

As used herein, the term "patient" can refer to any warm-blooded organism including, but not limited to, human beings, pigs, rats, mice, dogs, goats, sheep, horses, monkeys, apes, rabbits, cattle, etc. The term "emergency medical professional" can refer to anyone who provides care to a patient in an ambulatory setting or a hospital setting, including clinicians, nurses, emergency medical technicians, and the like.

It will be understood that, although the terms "first," "second," etc. may be used herein to describe various elements, these elements should not be limited by these terms. These terms are only used to distinguish one element from another. Thus, a "first" element discussed below could also be termed a "second" element without departing from the teachings of the present disclosure. The sequence of operations (or steps) is not limited to the order presented in the claims or figures unless specifically indicated otherwise.

In the context of the present disclosure, the singular forms "a," "an" and "the" can include the plural forms as well, unless the context clearly indicates otherwise. It will be further understood that the terms "comprises" and/or "comprising," as used herein, can specify the presence of stated features, steps, operations, elements, and/or components, but do not preclude the presence or addition of one or more other features, steps, operations, elements, components, and/or groups thereof. As used herein, the term "and/or" can include any and all combinations of one or more of the associated listed items.

It will be understood that when an element is referred to as being "on," "connected" to, "coupled" with, "contacting," etc., another element, it can be directly on, attached to, connected to, coupled with or contacting the other element or intervening elements may also be present. In contrast, when an element is referred to as being, for example, "directly on," "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

The present disclosure includes reference to block diagrams and/or flowchart illustrations of methods, apparatus (systems) and/or computer program products according to certain aspects of the disclosure. It is understood that each block of the block diagrams and/or flowchart illustrations, and combinations of blocks in the block diagrams and/or flowchart illustrations, can be implemented by computer program instructions. These computer program instructions may be provided to a processor of a general purpose computer, special purpose computer, and/or other programmable data processing apparatus to produce a machine, such that the instructions, which execute via the processor of the computer and/or other programmable data processing apparatus, create means for implementing the functions/acts specified in the block diagrams and/or flowchart block or blocks.

These computer program instructions may also be stored in a computer-readable memory that can direct a computer or other programmable data processing apparatus to function in a particular manner, such that the instructions stored in the computer-readable memory produce an article of manufacture including instructions, which implement the function/act specified in the block diagrams and/or flowchart block or blocks.

The computer program instructions may also be loaded onto a computer or other programmable data processing apparatus to cause a series of operational steps to be performed on the computer or other programmable apparatus to produce a computer-implemented process such that the instructions that execute on the computer or other programmable apparatus provide steps for implementing the functions/acts specified in the block diagrams and/or flowchart block or blocks.

Accordingly, the present disclosure may be embodied in hardware and/or in software (including firmware, resident software, microcode, etc.). Furthermore, aspects of the present disclosure may take the form of a computer program product on a computer-usable or computer-readable storage medium having computer-usable or computer-readable program code embodied in the medium for use by or in connection with an instruction execution system. A computer-usable or computer-readable medium may be any non-transitory medium that can contain or store the program for use by or in connection with the instruction or execution of a system, apparatus, or device.

The computer-usable or computer-readable medium may be, for example but not limited to, an electronic, magnetic, optical, electromagnetic, infrared, or semiconductor system, apparatus or device. More specific examples (a non-exhaustive list) of the computer-readable medium can include the following: a portable computer diskette; a random access memory; a read-only memory; an erasable programmable read-only memory (or Flash memory); and a portable compact disc read-only memory.

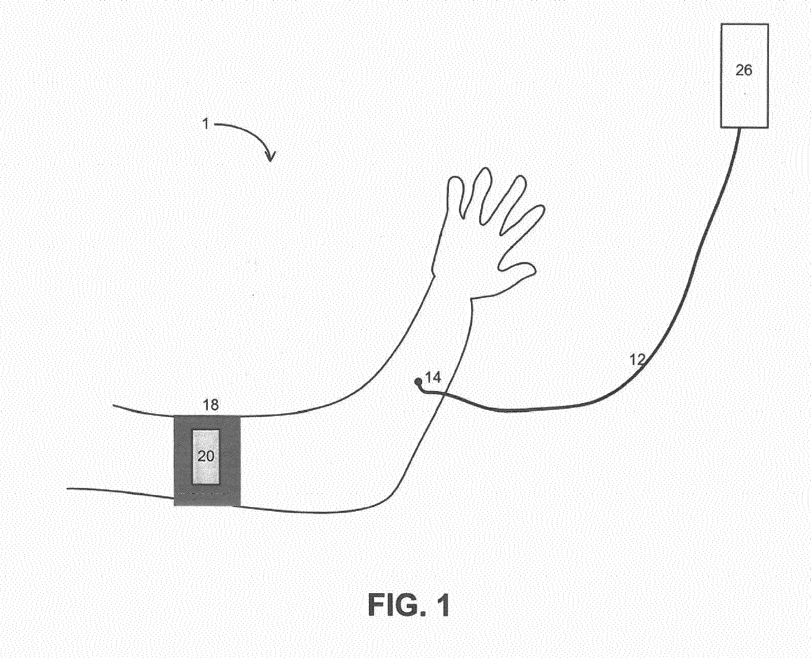

Referring now to FIG. 1, illustrated is a schematic illustration of an example hemodynamic resuscitation system 1 in accordance with one aspect of the present disclosure. System 1 can include a sensor coupled to an intravenous access device 12, capable of insertion into a peripheral vein of a patient. When inside the peripheral vein, the sensor can detect a peripheral venous pressure (PVP) and communicate an indication of the PVP via a communication device to a controller 20. The controller 20 can determine a hemodynamic value based on the PVP and develop a risk score based on the hemodynamic value. System 1 can be an open loop system that allows the emergency medical professional to act on the risk score with the appropriate action in his medical opinion. System 1 can, additionally or alternatively, be a closed loop system, where the controller 20 can alert a component of system 1 to deliver an amount (e.g., determined by the controller 20 based on the risk score and/or the hemodynamic parameter) of fluid to the patient from an external fluid source 26.

The system 1 can include an intravenous access device 12. The "intravenous access device" 12 can refer to a device that can be administered to a peripheral vein by the emergency medical professional, including, but not limited to, a catheter, a tubing set, a disposable intravenous tube, a needle and/or a valve.

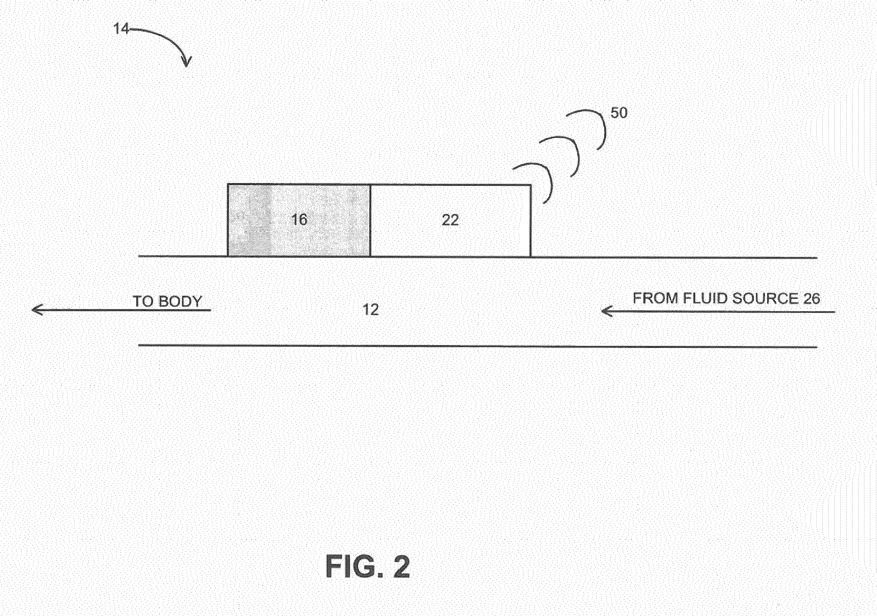

The intravenous access device 12 can be coupled to one or more sensor elements. The sensor elements can be administered to the peripheral vein with the intravenous access device 12. In other words, the sensor elements are capable of insertion into the peripheral vein (e.g., constructed from a biocompatible material). For example, the sensor elements can be located within at least a portion of the intravenous access device 12 that is inside the peripheral vein at point 14 of FIG. 1. FIG. 2 illustrates an example of the portion of the intravenous access device 12 that is within the peripheral vein at point 14.

The intravenous access device 12 as illustrated in FIG. 2 can include a sensor element 16 and a wireless communication device 22. It will be understood that the intravenous access device 12 can also include a controller device 20, such as a microcontroller, in addition to or instead of the wireless communication device 22. It will be understood that the controller device 20 can include the wireless communication device 22. The wireless communication device 22 can also be a wired communications device (e.g., providing a wired connection between the controller device 20 and the sensor element 16).

Each of the elements included with the intravenous access device 12 can be attached to the exterior of the intravenous access device 12, be included within the intravenous access device 12, or be configured in a different way within or near the intravenous access device so that the elements can be administered to the peripheral vein at substantially the same time as the intravenous access device 12.

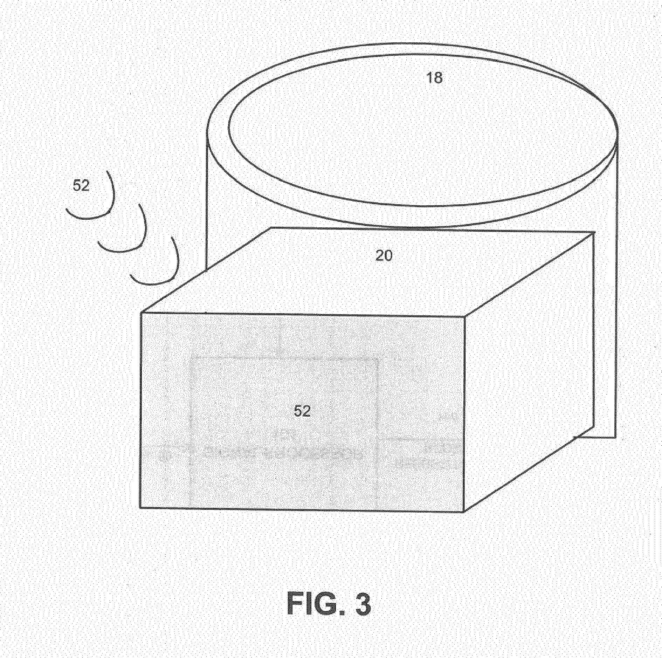

In an embodiment, the sensor element 16 is a pressure sensor element. The pressure sensor element can be configured to detect (or can be placed within the intravenous access device 12 in a way that it can detect) the PVP parameter within the peripheral vein. The PVP parameter can be detected when the vein is occluded (e.g., by occlusion device 18 in FIGs. 1 and 3).

The pressure sensor element can be a piezoelectric sensor, a capacitive sensor, a piezoresistive sensor, an electromagnetic sensor, a strain gauge, an optical sensor, a potentiometric sensor, a thermal sensor, a microelectromechanical system sensor (MEMS) sensor, or any other type of pressure sensor that can detect the PVP parameter within the peripheral vein. In addition to the pressure sensor element, the sensor element 16 can also include an element that can detect another parameter that can facilitate the hemodynamic resuscitation, including, but not limited to: a blood pressure parameter, a heart rate parameter, an electrocardiography parameter, a body impedance parameter, a blood oxygen saturation parameter, a body temperature parameter, a tonography parameter, and/or a plethysmography parameter.

The wireless communication device 22 can be a type of device that facilitates wireless communication of a signal 50, including the PVP value, to the controller 20. The wireless communication device 22 can be a device that can facilitate data exchange over short distances. In one example, the wireless communication device 22 can be a BLUETOOTH device that uses short-wavelength radio transmissions in the ISM band from 2400-2480 MHZ). The wireless communication device 22 can transmit a signal 50 that includes the PVP value to the controller 20.

The controller 20, as shown in FIG. 3, can receive signal 50 via wireless communication device. For example, the controller 20 can include a wireless communication device of the same type as wireless communication device 22 to facilitate reception of the transmitted signal 50. For example, if the wireless communication device 22 is a BLUETOOTH device, the wireless communication device within the controller is also a BLUETOOTH device. It will be understood that controller 20, additionally or alternatively, can receive signal 50 via a wired communication device.

The controller 20 can be in physical contact with an occlusion device 18. The physical contact can be a removable physical contact. The controller 20 need not be in physical contact with the occlusion device 18 and, instead, for example, can be in contact with another type of device that can be attached to the patient (e.g., a mat-like device that can store other patient essentials like extra tubing, tape, etc.). Additionally or alternatively, the controller 20 can be a stand-alone device (e.g., a box-type device) that can be otherwise in contact with or near the patient without making contact. The controller 20 can, additionally or alternatively, be in a location remote from the patient (e.g., in a control room).

As shown in FIGs. 1 and 3, the occlusion device 18 can be a cuff-type device that can inflate to facilitate the occlusion. However, the occlusion device can be any device that occludes a vein or multiple veins through methods including, but not limited to: external compression, intravenous balloon occlusion or cuff occlusion. The occlusion device 18 can be an independent device (operated or inflated independently from the controller 20) or occlusion device 18 can be operated or controlled by the controller 20 to occlude the vein upon a signal from the controller 20. Moreover, although FIG. 1 illustrates a left arm with the occlusion device 18 and the intravenous access device 12, it will be understood that the system 1 can be applied to either arm or either leg.

The controller 20 receives the signal 50 that includes the PVP value, and processes the signal 50 to determine a hemodynamic parameter. It will be understood that the controller can be a hardware controller (e.g., a microcontroller) that employs a processor and a non-transitory memory. Additionally, the controller 20 includes some form of power source.

The hemodynamic parameter can be a parameter that correlates to left ventricle end diastolic volume, stroke volume, cardiac output, tissue perfusion, or another parameter that relates to hemodynamic status. For example, the hemodynamic parameter can include one or more of a maximum occluded peripheral venous pressure (MOPVP), a cuff occluded rise of peripheral venous pressure (CORRP), and an integrated occluded peripheral venous pressure (IOPVP). The hemodynamic parameter can, additionally or alternatively, include one or more of a baseline (non-occluded) pressure reading, a rise time to 63% of the maximum occluded venous pressure, a mean square error, and a wavelet matching parameter. In other words, the hemodynamic parameter can be based on the PVP parameter included in signal 50 that can facilitate the generation of the resuscitation score.

The controller 50 can generate the resuscitation score based on the hemodynamic parameter. The resuscitation score can be displayed on a display device. An example of a display device is shown in FIG. 3, where the display device 52 can be a flexible display device, such as a flexible liquid crystal display (LCD) screen. However, the display device need not be coupled to the controller 20. The display device need only be able to receive a signal from the controller 20 that includes the resuscitation score and display the resuscitation score.

Generally, the resuscitation score is a value that the emergency medical professional can use to evaluate current intravascular volume status to determine if the patient is experiencing compensated shock or another application where hemodynamic status is important. For example, the resuscitation score can include a numerical value (e.g., the hemodynamic parameter or a function of the hemodynamic parameter). However, the resuscitation score need not be a number per se. The controller 20 can weigh the hemodynamic value against a threshold for compensative shock, and a resuscitation score that indicates compensated shock can be displayed as a flashing light, alarm, or any other indication designed to attract the attention of the emergency medical professional.

In an embodiment, based on the hemodynamic score, the emergency medical professional can determine an appropriate medical response (e.g., administering fluid from the external fluid source 26 through the intravenous access device 12 to the patient). In another embodiment, based on the hemodynamic score, the controller 20 can control fluid delivery from external fluid source 26 through the intravenous access device 12 to the patient. For example, the controller 20 can provide a signal that opens (or closes) a valve associated with the intravenous access device 12 to regulate the flow of fluid to the patient from the external fluid source 26. In another example, the controller 20 can provide an input signal to an external pump to modulate a fluid flow rate from the external fluid source 26. The fluid stored in the external fluid source can include, but is not limited to: a fluid solution (e.g., a saline solution), a blood product, a medication or a resuscitative solution.

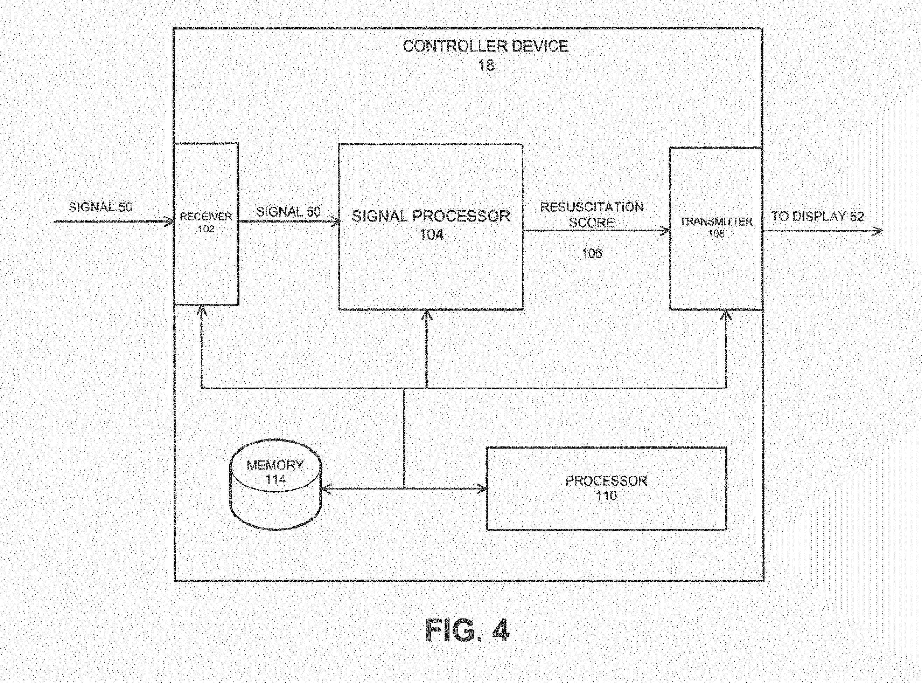

Referring now to Fig. 4. Illustrated is a schematic illustration of an example controller device 20 that can be utilized within the system 1 of FIG. 1. The controller device 20 can be integrated with other components of the system 1 or can be an independent device. In one example, controller 20 can be integrated with display device 52 and/or occlusion device 18. In another example, the controller 20 can be a standalone device.

The controller device 20 can include a processor 110 and a memory 114. The memory 114 can store instructions that can be executed by the processor 110 to facilitate hemodynamic resuscitation.

The controller device 20 can include a receiver 102, a signal processor 104 and a transmitter 108. The signal processor 104 can be independent from processor 110, but can also be a part of processor 110. The receiver 102 and transmitter 104 can be components of a wireless communications device and/or a wired communications device.

The receiver 102 can receive the signal 50 that includes data related to the PVP recorded by the pressure sensor within the intravenous access device. The signal processor 104 can process the signal 50 to achieve a hemodynamic parameter. The hemodynamic parameter can be any parameter that correlates to left ventricle end diastolic volume or stroke volume, cardiac output, tissue perfusion or another parameter that relates to hemodynamic status. For example, the hemodynamic parameter can be one or more of a maximum occluded peripheral venous pressure (MOPVP), a cuff occluded rise of peripheral venous pressure (CORRP), and an integrated occluded peripheral venous pressure (IOPVP). The hemodynamic parameter can, additionally or alternatively, be one or more of a baseline (non-occluded) pressure reading, a rise time to 63% of the maximum occluded venous pressure, a mean square error, and a wavelet matching parameter. In other words, the hemodynamic parameter can be a parameter included in signal 50 that can facilitate the generation of an accurate resuscitation score 106.

The signal processor 104 can generate the resuscitation score 106 based on the hemodynamic parameter. The signal processor 104 can apply a weighting to different values within the hemodynamic parameter and/or compare wavelets within the hemodynamic parameter to a template wavelet function to achieve the resuscitation score 106. The transmitter 108 can transmit the resuscitation score 106 to a display 52. The display 52 can display a number value for the resuscitation score, play an audio sound or alarm when the resuscitation score falls below a threshold value indicating compensative shock, or display an animation or color change when the resuscitation score falls below a threshold value indicating compensative shock.

in an example, the transmitter 108 can transmit a signal to a component of the intravenous access device to allow a certain amount of fluid to be delivered to the patient based on the resuscitation score. As an example, the component of the intravenous access device can be a valve that is signaled to open or close to allow or prohibit passive flow of fluid. In another example, the component of the intravenous access device can be a pump that is signaled to actively pump a certain amount of fluid to the patient.

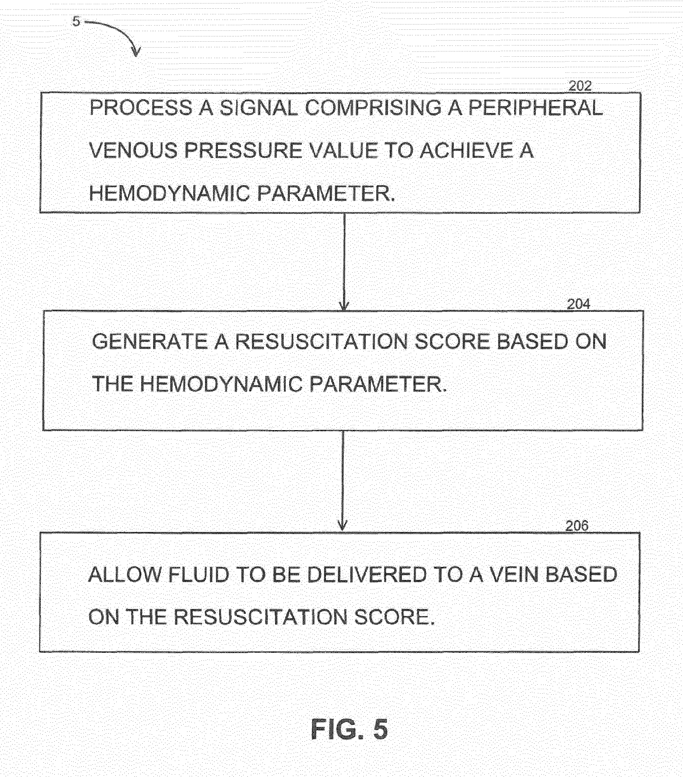

In view of the foregoing structural and functional features described above, a method in accordance with various aspects of the present invention will be better appreciated with reference to FIG. 5. While, for purposes of simplicity of explanation, the method of FIG. 5 is shown and described as executing serially, it is to be understood and appreciated that the present invention is not limited by the illustrated order, as some aspects could, in accordance with the present invention, occur in different orders and/or concurrently with other aspects from that shown and described herein. Moreover, not all illustrated features may be required to implement a methodology in accordance with an aspect of the present invention. It will be appreciated that some or all of each of these methods can be implemented as machine-executable instructions stored on a non-transitory computer readable device (e.g., memory 118). The instructions can be executed by a processor (e.g., processor 116) to facilitate the performance of operations of the method.

FIG. 5 illustrates an example of a method 5 for hemodynamic resuscitation (e.g., to minimize symptoms of compensated shock in a patient). At 200, a signal (e.g., signal 50) that includes a PVP value (e.g., recorded by the pressure sensor) is processed (e.g., by signal processor 104) to achieve a hemodynamic parameter. At 210, a resuscitation score (e.g., resuscitation score 106) is generated (e.g., by signal processor 104) based on the hemodynamic parameter. At 220, fluid (e.g., from external fluid source 24) is allowed to be delivered to a peripheral vein (e.g., based on a signal from controller 20 or via a decision by the emergency medical professional) based on the resuscitation score.

From the above description, those skilled in the art will perceive improvements, changes and modifications.

## Claims

1. A hemodynamic resuscitation system comprising:
an occlusion device (18) configured to occlude a peripheral vein;
an intravenous access device (12) having a pressure sensor element (16) configured to detect a peripheral venous pressure value in response to the occlusion of a peripheral vein by the occlusion device (18) and to interface with an external fluid source (26) for fluid delivery to the vein; and
a controller device (20), connected to the pressure sensor element (16) and the external fluid source (26), configured to:
receive a signal (50) comprising data related to the peripheral venous pressure value, process the signal to determine a hemodynamic parameter by comparing wavelets within the signal to a template wavelet function, and generate a resuscitation score (106) based on the hemodynamic parameter,
wherein an amount of the resuscitative fluid is delivered to the peripheral vein from the fluid source through the intravenous access device based on the resuscitation score.

2. The system of claim 1, wherein the intravenous access device (12) comprises a valve configured to allow the fluid delivery from the fluid source (26) when controlled by the controller device (20).

3. The system of claim 1, wherein the external fluid source (26) stores a fluid, a blood product, a medication, or a resuscitative solution.

4. The system of claim 1, wherein the hemodynamic parameter comprises at least one of a maximum occluded peripheral venous pressure, a cuff occluded rise of peripheral venous pressure, and an integrated occluded peripheral venous pressure.

5. The system of claim 1, wherein the pressure sensor comprises a first wireless communication device (22) configured to transmit the signal to the controller device (20) that comprises a second wireless communication device (22).

6. The system of claim 1, wherein the intravenous access device (12) comprises a disposable intravenous tube, a needle, a catheter, or a valve.

7. The system of claim 1, wherein the controller device (20) comprises a display device (52) configured to display the resuscitation score (106).

8. The system of claim 1, wherein the pressure sensor comprises a piezoelectric sensor, a capacitive sensor, a piezoresistive sensor, an electromagnetic sensor, a strain gauge, an optical sensor, a potentiometric sensor, or a thermal sensor.

9. The system of claim 1, wherein the controller device (20) is physically coupled to the occlusion device (18).

10. The system of claim 1, wherein the occlusion device (18) is a cuff-type device configured to occlude the peripheral vein by cuff occlusion.

11. A non-transitory computer-readable device storing instructions executable by the controller device (20) of claim 1 to perform operations that facilitate hemodynamic resuscitation, the operations comprising:
processing a signal comprising data related to a peripheral venous pressure value detected by a pressure sensor within an intravenous access device to achieve a hemodynamic parameter by comparing wavelets within the signal to a template wavelet function;
generating a resuscitation score based on the hemodynamic parameter; and
signaling a component of the intravenous access device to allow an amount of fluid to be delivered from an external fluid source to the vein, based on the resuscitation score.

12. The non-transitory computer-readable device of claim 11, wherein the resuscitation score is additionally based on applying a weighting function to the hemodynamic parameter.

13. The non-transitory computer-readable device of claim 11, wherein the hemodynamic parameter comprises at least one of a maximum occluded peripheral venous pressure, a cuff occluded rise of peripheral venous pressure, and an integrated occluded peripheral venous pressure.

## Patentansprüche

1. Hämodynamisches Wiederbelebungssystem, das Folgendes umfasst:
eine Okklusionsvorrichtung (18), die zum Verschließen einer peripheren Vene konfiguriert ist;
eine intravenöse Zugangsvorrichtung (12), die ein Drucksensorelement (16) aufweist, zur Detektion eines Werts des peripheren Venendrucks als Reaktion auf den Verschluss einer peripheren Vene durch die Okklusionsvorrichtung (18) und zur Verbindung mit einer externen Flüssigkeitsquelle (26) für eine Flüssigkeitsabgabe an die Vene konfiguriert ist; und
eine Regelvorrichtung (20), die mit dem Drucksensorelement (16) und der externen Flüssigkeitsquelle (26) verbunden ist, die für Folgendes konfiguriert ist:
Empfangen eines Signals (50), das Daten bezüglich des Werts des peripheren Venendrucks umfasst, Verarbeiten des Signals, um einen hämodynamischen Parameter durch Vergleich von Wavelets innerhalb des Signals mit einer Wavelet-Template-Funktion zu bestimmen, und Erzeugen eines Wiederbelebungs-Scores (106) basierend auf dem hämodynamischen Parameter,
wobei eine Menge der Wiederbelebungsflüssigkeit an die peripheren Vene aus der Flüssigkeitsquelle durch die intravenöse Zugangsvorrichtung basierend auf dem Wiederbelebungs-Score abgegeben wird.

2. System nach Anspruch 1, wobei die intravenöse Zugangsvorrichtung (12) ein Ventil umfasst, das konfiguriert ist, um die Flüssigkeitsabgabe aus der Flüssigkeitsquelle (26) zu ermöglichen, wenn es durch die Regelvorrichtung (20) geregelt wird.

3. System nach Anspruch 1, wobei die externe Flüssigkeitsquelle (26) eine Flüssigkeit, ein Blutprodukt, ein Medikament oder eine Wiederbelebungslösung speichert.

4. System nach Anspruch 1, wobei der hämodynamische Parameter mindestens eines von Folgenden umfasst: einen maximalen peripheren Venendruck bei Verschluss, einen Anstieg eines peripheren Venendrucks bei Verschluss durch eine Manschette und einen peripheren Venendruck bei Verschluss bei Integration.

5. System nach Anspruch 1, wobei der Drucksensor eine erste drahtlose Kommunikationsvorrichtung (22) umfasst, die zur Übertragung des Signals zur Regelvorrichtung (20) konfiguriert ist, die eine zweite drahtlose Kommunikationsvorrichtung (22) umfasst.

6. System nach Anspruch 1, wobei die intravenöse Zugangsvorrichtung (12) einen intravenösen Einwegschlauch, eine Nadel, einen Katheter oder ein Ventil umfasst.

7. System nach Anspruch 1, wobei die Regelvorrichtung (20) eine Anzeigevorrichtung (52) umfasst, die zur Anzeige des Wiederbelebungs-Scores (106) konfiguriert ist.

8. System nach Anspruch 1, wobei der Drucksensor Folgendes umfasst: einen piezoelektrischen Sensor, einen kapazitiven Sensor, einen piezoresistiven Sensor, einen elektromagnetischen Sensor, einen Dehnungsmessstreifen, einen optischen Sensor, einen potentiometrischen Sensor oder einen thermischen Sensor.

9. System nach Anspruch 1, wobei die Regelvorrichtung (20) mit der Okklusionsvorrichtung (18) physisch verbunden ist.

10. System nach Anspruch 1, wobei die Okklusionsvorrichtung (18) eine manschettenartige Vorrichtung ist, die zum Verschluss der peripheren Vene durch Manschettenverschluss konfiguriert ist.

11. Nichttransitorische computerlesbare Vorrichtung, die Anweisungen speichert, die durch die Regelvorrichtung (20) nach Anspruch 1 ausführbar sind, um Vorgänge auszuführen, die eine hämodynamische Wiederbelebung ermöglichen, wobei die Vorgänge Folgendes umfassen:
Verarbeiten eines Signals, das Daten bezüglich eines Werts des peripheren Venendrucks umfasst, der von einem Drucksensor innerhalb einer intravenösen Zugangsvorrichtung detektiert wurde, um einen hämodynamischen Parameter durch Vergleich von Wavelets innerhalb des Signals mit einer Wavelet-Template-Funktion zu erhalten;
Erzeugen eines Wiederbelebungs-Scores basierend auf dem hämodynamischen Parameter; und
Signalisieren an eine Komponente der intravenösen Zugangsvorrichtung, um zu ermöglichen, dass eine Menge an Flüssigkeit aus einer externen Flüssigkeitsquelle an die Vene, basierend auf dem Wiederbelebungs-Score, abgegeben wird.

12. Nichttransitorische computerlesbare Vorrichtung nach Anspruch 11, wobei der Wiederbelebungs-Score zusätzlich auf der Anwendung einer Gewichtungsfunktion auf den hämodynamischen Parameter basiert.

13. Nichttransitorische computerlesbare Vorrichtung nach Anspruch 11,
wobei der hämodynamische Parameter mindestens eines von Folgenden umfasst: einen maximalen peripheren Venendruck bei Verschluss, einen Anstieg eines peripheren Venendrucks bei Verschluss durch eine Manschette und einen peripheren Venendruck bei Verschluss bei Integration.

## Revendications

1. Un système de réanimation hémodynamique comprenant :
Un dispositif d'occlusion (18) configuré pour obstruer une veine périphérique ;
Un dispositif d'accès intraveineux (12) doté d'un élément capteur de pression (16) configuré pour détecter une valeur de pression veineuse périphérique en réponse à l'occlusion d'une veine périphérique par le dispositif d'occlusion (18) et pour interagir avec une source de fluide externe (26) permettant l'administration de fluide dans la veine ; et
Un contrôleur (20), connecté à l'élément capteur de pression (16) et à la source de fluide externe (26), configuré pour :
Recevoir un signal (50) comprenant des données relatives à la valeur de la pression veineuse périphérique, traiter le signal pour déterminer un paramètre hémodynamique en comparant les ondelettes du signal à une fonction modèle d'ondelettes, et générer un score de réanimation (106) en fonction du paramètre hémodynamique,
Dans lequel une quantité de fluide de réanimation est envoyée dans la veine périphérique à partir de la source de fluide par le biais du dispositif d'accès intraveineux en fonction du score de réanimation.

2. Le système de la revendication 1, dans lequel le dispositif d'accès intraveineux (12) comprend une soupape configurée pour permettre l'administration de fluide à partir de la source de fluide (26) lorsqu'il est contrôlé par le contrôleur (20).

3. Le système de la revendication 1, dans lequel la source de fluide externe (26) stocke un fluide, un produit sanguin, un médicament, ou une solution de réanimation.

4. Le système de la revendication 1, dans lequel le paramètre hémodynamique comprend au moins une pression veineuse périphérique d'un maximum combiné de pressions veineuses périphériques mesurées par occlusion, une élévation de la pression prise par un brassard d'occlusion veineuse périphérique, et une pression veineuse périphérique intégrée mesurée par occlusion.

5. Le système de la revendication 1, dans lequel le capteur de pression est composé d'un premier dispositif de communication sans fil (22) configuré pour transmettre le signal au contrôleur (20) qui comprend un deuxième dispositif de communication sans fil (22).

6. Le système de la revendication 1, dans lequel le dispositif d'accès intraveineux périphérique (12) comprend une canule intraveineuse jetable, une aiguille, un cathéter, ou une soupape.

7. Le système de la revendication 1, dans lequel le contrôleur (20) comprend un dispositif d'affichage (52) configuré pour afficher le score de réanimation (106).

8. Le système de la revendication 1, dans lequel le capteur de pression est composé d'un capteur piézoélectrique, d'un capteur capacitif, d'un capteur piézorésistif, d'un capteur électromagnétique, d'une jauge de contrainte, d'un capteur optique, d'un capteur potentiométrique, ou d'un capteur thermique.

9. Le système de la revendication 1, dans lequel le contrôleur (20) est physiquement relié au dispositif d'occlusion (18).

10. Le système de la revendication 1, dans lequel le dispositif d'occlusion (18) est un dispositif de type brassard configuré pour obstruer la veine périphérique en la comprimant.

11. Un dispositif non transitoire lisible par ordinateur stockant des consignes exécutables par le contrôleur (20) de la revendication 1 pour effectuer des opérations qui facilitent la réanimation hémodynamique, ces opérations comprenant :
Le traitement d'un signal comprenant des données relatives à une valeur de pression veineuse périphérique détectée par un capteur de pression à l'intérieur d'un dispositif d'accès intraveineux pour déterminer un paramètre hémodynamique en comparant les ondelettes du signal à une fonction modèle d'ondelettes ;
La génération d'un score de réanimation en fonction des paramètres hémodynamiques et
L'envoi d'un signal à un composant du dispositif d'accès intraveineux pour permettre à une quantité de fluide d'être envoyée à partir d'une source de fluide externe à la veine, en fonction du score de réanimation.

12. Le dispositif non transitoire lisible par ordinateur de la revendication 11, dans lequel le score de réanimation est de plus basé sur l'application d'une fonction de pondération au paramètre hémodynamique.

13. Le dispositif non transitoire lisible par ordinateur de la revendication 11, dans lequel le paramètre hémodynamique comprend au moins une pression veineuse périphérique d'un maximum combiné de pressions veineuses périphériques mesurées par occlusion, une élévation de la pression prise par un brassard d'occlusion veineuse périphérique, et une pression veineuse périphérique intégrée mesurée par occlusion.
